(19) Europäisches Patentamt
European Patent Office
Office européen des brevets

(11) Publication number: 0 340 849 A2

# (12) EUROPEAN PATENT APPLICATION

(21) Application number: 89201079.4

(22) Date of filing: 25.04.89

(51) Int. Cl.4: C07D 493/22 , C07H 19/01 , A61K 31/365 , A61K 31/70 , A01N 43/90 , //(C07D493/22, 313:00,311:00,311:00,307:00)

(30) Priority: 02.05.88 US 189445

(43) Date of publication of application: 08.11.89 Bulletin 89/45

(84) Designated Contracting States: AT BE CH DE ES FR GB IT LI LU NL SE

(71) Applicant: MERCK & CO. INC.
126, East Lincoln Avenue P.O. Box 2000
Rahway New Jersey 07065-0900(US)

(72) Inventor: Eskola, Philip
2815 Constitution Way
Wall Township New Jersey 07719(US)
Inventor: Fisher, Michael H.
RD1 Old York Road
Box 302, Ringoes New Jersey 08551(US)
Inventor: Mrozik, Helmut
159 Idelbrook Lane
Matawan New Jersey 07747(US)

(74) Representative: Hesketh, Alan, Dr.
European Patent Department Merck & Co., Inc. Terlings Park Eastwick Road
Harlow Essex, CM20 2QR(GB)

## (54) Fluoroavermectin and fluoromilbemycin derivatives with antiparasitic and pesticidal properties.

(57) Novel avermectin and milbemycin compounds, wherein at least one of the 4a, 5 or 4″ positions are substituted with a fluoro groups, are prepared by replacement of hydroxy or other suitable leaving group of avermectin and milbemycin compounds with fluorine. The fluoroavermectin and fluoromilbemycin compounds have utility as anti-parasitic agents and as highly potent insecticides against agricultural pests.

EP 0 340 849 A2

# FLUOROAVERMECTIN AND FLUOROMILBEMYCIN DERIVATIVES WITH ANTIPARASITIC AND PESTICIDAL PROPERTIES

## BACKGROUND OF THE INVENTION

The term avermectin (previously referred to as C-076) is used to describe a series of compounds isolated from the fermentation broth of an avermectin producing strain of Streptomyces avermitilis and derivatives thereof. The morphological characteristics of the culture are completely described in U.S. Pat. No. 4,310,519. The avermectin compounds are a series of macrolides, each of which is substituted at the 13-position with a 4′-(α-L-oleandrosyl)-α-L-oleandrose group. The avermectin compounds and the instant derivatives thereof have a high degree of anthelmintic and anti-parasitic activity.

The avermectin series of compounds isolated from the fermentation broth have the following structure:

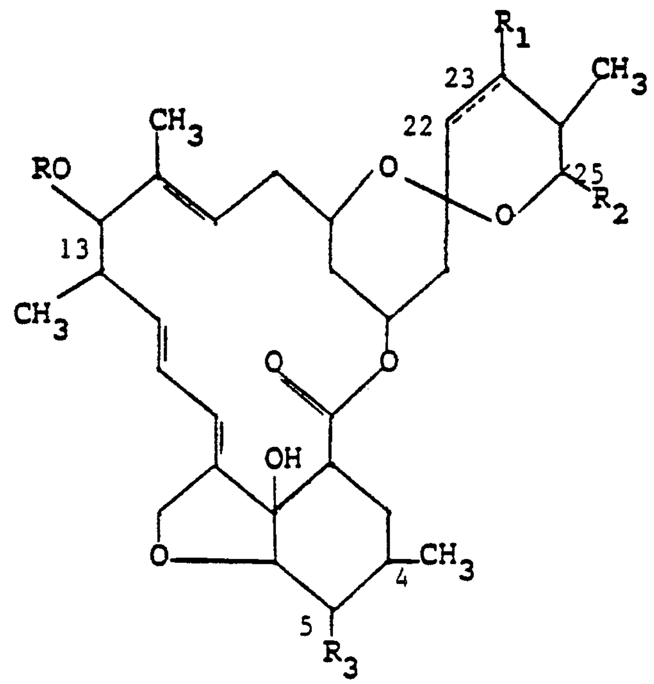

wherein R is the 4′-(α-L-oleandrosyl)-α-L-oleardrose group of the structure:

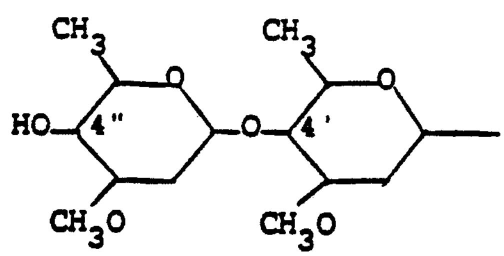

and wherein the broken lines indicate single or double bonds; $R_1$ is hydroxy and is present only when said 22,23 broken line indicates a single bond; $R_2$ is iso-propyl or sec-butyl; and

$R_3$ is methoxy or hydroxy.

There are eight different major avermectin natural product compounds and they are given the designations A1a, A1b, A2a, A2b, B1a, B1b, B2a and B2b based upon the structure of the individual compounds.

In structural formula Ia above, the individual avermectin compounds are as set forth below wherein the R group is 4′-(α-L-oleandrosyl)-α-L-oleandrose:

| | 22,23 | $R_1$ | $R_2$ | $R_3$ |
|---|---|---|---|---|
| A1a | Double Bond | - | sec-butyl | $-OCH_3$ |
| A1b | Double Bond | - | iso-propyl | $-OCH_3$ |
| A2a | Single Bond | -OH | sec-butyl | $-OCH_3$ |
| A2b | Single Bond | -OH | iso-propyl | $-OCH_3$ |
| B1a | Double Bond | - | sec-butyl | -OH |
| B1b | Double Bond | - | iso-propyl | -OH |
| B2a | Single Bond | -OH | sec-butyl | -OH |
| B2b | Single Bond | -OH | iso-propyl | -OH |

The avermectin compounds are generally isolated as mixtures of a and b components which differ only in the nature of the $R_2$ substituent. These minor structural differences have been found to have very little effect on the isolation procedures, chemical reactivity and biological activity of such compounds.

Milbemycin compounds are similar to the above avermectin compounds in that the 16-membered macro cyclic ring is present. However, such compounds have no substitution at the 13-position and have a methyl, ethyl or a $C_{3-6}$ branched alkyl group at the 25-position (i.e., the position of the $R_2$ group in the above structural formula I). The anthelmintic LL-F28249 compounds, which have a branched butyl, pentyl or hexyl group containing one unsaturation at the 26-position and lack the 13-disaccharide group, are also starting materials for the instant compounds. They are disclosed in European Patent Application No. 85106844.5. To the extent that such milbemycin compounds have hydroxy groups or can be converted to compounds with hydroxy groups which can then be substituted with the instant fluoro groups, they are to be construed as being within the ambit of this invention. Such milbemycin compounds and the fermentation conditions used to prepare them are described in U.S. Pat. No. 3,950,360. In addition, 13-deoxyavermectin aglycones are prepared synthetically from the avermectin natural products and are disclosed in U.S. Pat. Nos. Re 32006 and Re 32034. Such compounds are very similar to the milbemycins differing from some of the milbemycins in having an isopropyl or sec-butyl rather than a methyl or ethyl group at the 25-position.

U.S. Pat. No. 4,469,682 discloses phosphate esters of avermectin and milbemycin which are stated to have improved water solubility over the parent avermectin and milbemycin compounds and which are useful as anti-parasitic agents and insecticides.

U.S. Pat. No. 4,547,491 discloses avermectin and milbemycin compound having the 8a position oxidized to a ketone group. U.S. Pat. No. 4,579,864 discloses a ketone group, imine group or an amino group in the 13-position. U.S. Pat. No. 4,622,313 discloses O-sulfate derivatives of avermectin and compounds.

The compounds of the present invention differ from other avermectin and milbemycin derivatives by the presence of a fluoro group at either the 4a, 5 or 4″ positions.

## SUMMARY OF THE INVENTION

The instant invention is concerned with certain derivatives of avermectin and milbemycin compounds wherein a fluorine group is introduced at the 4a, 5 or 4″ positions. Thus, it is an object of the instant invention to describe such avermectin and milbemycin derivatives. A further object is to describe processes for the preparation of such compounds. A still further object is to describe the uses of such compounds as antiparasitic agents. An additional object is to describe the uses of such compound as insecticidal agents for the treatment of household and agricultural insect pests. Still further objects will become apparent from reading of the following description.

## DESCRIPTION OF THE INVENTION

The compounds of the instant invention have the following structural formula:

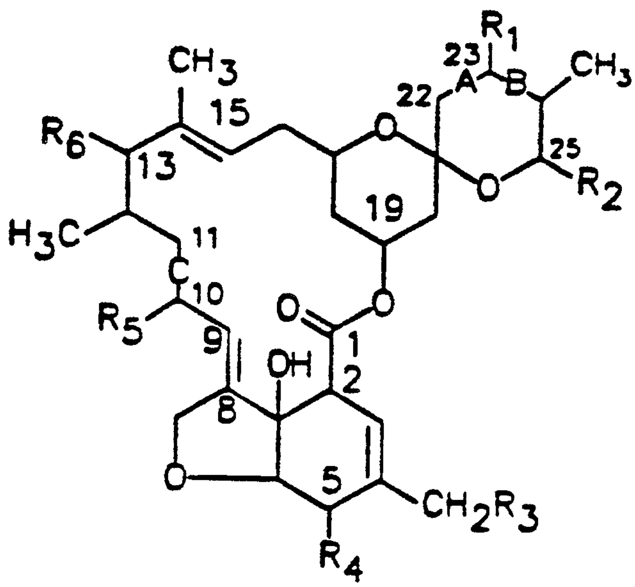

wherein:

A, B and C indicate single or double bonds.

$R_1$ is hydrogen, or hydroxy when A and B are single bonds;

$R_2$ is $CH_3$, $C_2H_5$, $CH(CH_3)_2$, $CH(CH_3(C_2H_5$, $C(CH_3)=CHCH_3$, $C(CH_3)=CHC_2H_5$, $C(CH_3)=CHCH(CH_3)_2$;

$R_3$ is hydrogen or fluorine;

$R_4$ is hydroxy or fluorine;

$R_5$ is hydrogen, or fluorine when C is a single bond;

$R_6$ is hydrogen, hydroxy, chlorine, fluorine or

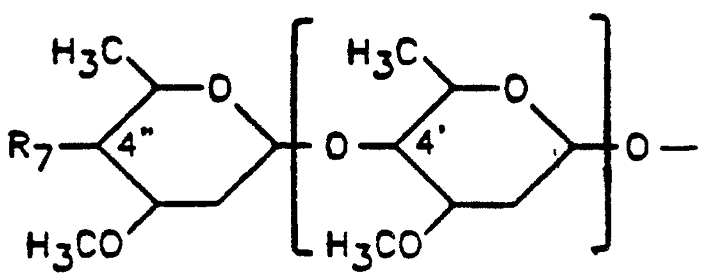

n=1 or 0

wherein

$R_7$ is hydroxy, fluorine, $NH_2$, NH-alkyl, N,N-dialkyl, NHCO-alkyl, N(alkyl)CO-alkyl and n is 0 or 1, provided that at least one group of $R_3$, $R_4$ or $R_7$ is a fluoro group.

The term "alkyl" when used in the instant application is intended to represent those groups containing either straight or branched chains, which have from 1-6 carbon atoms, or cycloalkyl groups of from 3 to 6 carbon atoms. Examples of such alkyl groups are methyl, ethyl, propyl, iso-propyl, butyl, sec-butyl, pentyl, hexyl and the like.

Examples of preferred compounds of the instant invention and their physiologically acceptable salts are:

5,13-Di-deoxy-5-fluoroavermectin B1a/B1b aglycone;

5-Deoxy-5-fluoromilbemycins $\alpha_1$ and/or $\alpha_3$;

5,23-Di-deoxy-23,24-dehydro-5-fluoro-anthelmintic-F-28249-$\alpha$;

5-Deoxy-5-fluoroavermectin B1a/B1b;

4″-Deoxy-4″-fluoroavermectin B1a/B1b, isomer A;

4″-Deoxy-4″-fluoroavermectin B1a/B1b, isomer B;

4a-Fluoro-13-deoxyavermectin B1a/B1b aglycone;

4a-Fluoro-22,23-dihydroavermectin B1a/B1b;

4a-Fluoro-13-deoxy-22,23-dihydroavermectin B1a/B1b aglycone;

4a-Fluoromilbemycin $\alpha_1$ and/or $\alpha_3$;

4a-Fluoro-anthelmintic-F-28249-$\alpha$;

4″-Deoxy-10,11-dihydro-4″,10-difluoroavermectin B1a/B1b, 4″-isomer A;
4″-Deoxy-10,11-dihydro-4″,10 difluoroavermectin B1a/B1b, 4″-isomer B;
5,13-Difluoro-22,23-dihydroavermectin B1a/B1b aglycone;
5-Deoxy-5-fluoro-22,23-dihydroavermectin B1a/B1b;
4a-Fluoro-4″-deoxy-4″-epi-methylaminoavermectin B1a/B1b;
5-Deoxy-5-fluoro-4″-oxoavermectin B1a/B1b;
5-Fluoro-4″,5-di-deoxy-4″-epi-methylaminoavermectin B1a/B1b;
23,24-Dehydro-5,23-di-deoxy-5-fluoroavermectin B2a/B2b;
4″-Fluoroavermectin B2a/B2b;
23,24-Dehydro-4″-23-di-deoxy-4″-fluoroavermectin B2a/B2b;
23,24-Dehydro-5-fluoro-5,13,23-trideoxyavermectin B2a/B2b aglycone; and
4″-Deoxy-22,23-dihydro-4″-fluoroavermectin B1a/B1b.

The fluoro derivatives of this invention are prepared by treating an avermectin compound having a hydroxy or other suitable leaving group in the desired position with diethylaminosulfur trifluoride (DAST). The methods of treatment may differ depending on the desired reaction, including a solution of DAST in $CH_2Cl_2$ and excess diethylaminosulfur trifluoride reagent (DAST).

## DISCUSSION OF UTILITY

The novel compounds of this invention have parasiticidal activity as anthelmintics, ectoparasiticides, insecticides and acaricides, in human and animal health and in agriculture.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxcyuris, Ancylostoma, Uncinaria, Toxascaris and Parascaris. Certain of these, such as Nematodirus, Cooperia and Oesphagostomum attack primarily the intestinal tract while others, such as Haemonchus and Ostertagia, are more prevalent in the stomach while still others such as Dictyocaulus are found in the lungs. Still other parasites may be located in other tissues and organs of the body such as the heart and blood vessels, subcutaneous and lymphatic tissue and the like. The parasitic infections known as helminthiases lead to anemia, malnutrition, weakness, weight loss, severe damage to the walls of the intestinal tract and other tissues and organs and, if left untreated, may result in death of the infected host. The substituted avermectin compounds of this invention have activity against these parasites, and in addition are also active against Dirofilaria in dogs, Namatospiroides, Syphacia, Aspiculuris in rodents, arthropod ectoparasites of animals and birds such as ticks, mites, lice, fleas, blowfly, in sheep Lucilia sp., biting insects and such migrating dipterous larvae as Hypoderma sp. in cattle, Gastrophilus in horses, and Cuterebra sp. in rodents.

The instant compounds are also useful against parasites which infect humans. The most common genera of parasties of the gastro-intestinal tract of man are Ancylostoma, Necator, Ascaris, Strongyloides, Trichinella, Capillaria, Trichuris, and Enterobius. Other medically important genera of parasites which are found in the blood or other tissues and organs outside the gastrointestinal tract are the filiarial worms such as Wuchereria, Brugia, Onchocerca and Loa, Dracunculus and extra intestinal stages of the intestinal worms Strongyloides and Trichinella. The compounds are also of value against arthropods parasitizing man, biting insects and other dipterous pests causing annoyance to man.

The compounds are also active against household pests such as the cockroach, Blatella sp., clothes moth, Tineola sp., carpet beetle, Attagenus sp., and the housefly Musca domestica.

The compounds are also useful against insect pests of stored grains such as Tribolium sp., Tenebrio sp. and of agricultural plants such as two spotted spider mites, (Tetranychus sp.), aphids, (Acyrthiosiphon sp.); against migratory orthopterans such as locusts and immature stages of insects living on plant tissue. The compounds are useful as a nematocide for the control of soil nematodes and plant parasites such as Meloidogyne spp. which may be of importance in agriculture. The compounds are active against other plant pests such as the southern army worm and Mexican bean beetle larvae.

These compounds may be administered orally in a unit dosage form such as a capsule, bolus or tablet,

or as a liquid drench where used as an anthelmintic in mammals. The drench is normally a solution, suspension or dispersion of the active ingredient usually in water together with a suspending agent such as bentonite and a wetting agent or like excipient. Generally, the drenches also contain an antifoaming agent. Drench formulations generally contains from about 0.001 to 0.5% by weight of the active compound. Preferred drench formulations may contain from 0.01 to 0.1% by weight. The capsules and boluses comprise the active ingredient admixed with a carrier vehicle such as starch, talc, magnesium stearate, or di-calcium phosphate.

Where it is desired to administer the avermectin derivatives in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with respect to their total weight and content of the antiparasitic agent depending upon factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or optionally fed separately. Alternatively, the antiparasitic compounds of our invention may be administered to animals parenterally, for example, by intraruminal, intramuscular, intratracheal, or subcutaneous injection in which event the active ingredient is dissolved or dispersed in a liquid carrier vehicle. For parenteral administration, the active material is suitably admixed with an acceptable vehicle, preferably of the vegetable oil variety such as peanut oil, cotton seed oil and the like. Other parenteral vehicles such as organic preparation using solketal, glycerol formal, and aqueous parenteral formulations are also used. The active avermectin compound or compounds are dissolved or suspended in the parenteral formulation for administration; such formulations generally contain from 0.005 to 5% by weight of the active compound.

Although the antiparasitic agents of this invention find their primary use in the treatment and/or prevention of helminthiasis, they are also useful in the prevention and treatment of diseases caused by other parasites, for example, arthropod parasites such as ticks, lice, fleas, mites and other biting insects in domesticated animals and poultry. They are also effective in treatment of parasitic diseases that occur in other animals including humans. The optimum amount to be employed for best results will, of course, depend upon the particular compound employed, the species of animal to be treated and the type and severity of parasitic infection or infestation. Generally good results are obtained with our novel compounds by administering about 0.001 to 10 mg of drug per kg of animal body weight, such total dose being given at one time or in divided doses over a relatively short period of time such as 1-5 days. With the preferred compounds of the invention, control of such parasites is obtained in animals by administering from about 0.025 to 1 mg per kg of body weight in a single dose. Repeat treatments are given as required to combat re-infections and are dependent upon the species of parasite and the husbandry techniques being employed. The techniques for administering these materials to animals are known to those skilled in the veterinary field.

Additionally, as an agent against external parasites, compounds of this invention may be prepared in formulations for topical applications, such as cremes, ointments, sprays, aerosols, dips, etc. The techniques for such administration to animals are likewise known to those skilled in the veterinary field.

When the compounds described herein are administered as a component of the feed of the animals, or dissolved or suspended in the drinking water, compositions are provided in which the active compound or compounds are intimately dispersed in an inert carrier or diluent. An inert carrier is one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like. The active avermectin compounds are intimately dispersed throughout the carrier by methods such as grinding, stirring, milling or tumbling. Compositions containing from about 0.005 to 2.0% by weight of the active compound are particularly suitable as feed premixes. Feed supplements, which are fed directly to the animal, contain from about 0.0002 to 0.3% by weight of the active compounds.

Such supplements are added to the animal feed in an amount to give the finished feed the concentration of active compound desired for the treatment and control of parastic diseases. Although the desired concentration of active compound will vary depending upon the factors previously mentioned as

well as upon the particular avermectin derivative employed, the compounds of this invention are usually fed at concentrations of between 0.00001 to 0.002% in the feed in order to achieve the desired antiparasitic result.

The avermectin compounds of this invention are also useful in combatting agricultural pests that inflict damage upon crops while they are growing or while in storage. The compounds are applied using known techniques as sprays, dusts, emulsions and the like, to the growing or stored crops to effect protection from such agricultural pests.

In using the compounds of this invention, the individual substituted avermectin components may be prepared and used in that form. Alternatively, mixtures of two or more of the individual avermectin components may be used, as well as mixtures of the parent avermectin compounds, other avermectin compounds or other active compounds not related to avermectin, with the compounds of this invention.

In the isolation of the avermectin compounds, which serve as starting materials for the instant processes, from the fermentation broth, the various avermectin compounds will be found to have been prepared in unequal amounts. In particular an "a" series compound will be prepared in a higher proportion than the corresponding "b" series compound.

The "b" compounds, those with a 25-isopropyl group, need not be separated from the corresponding "a" compound with a 25-sec-butyl group and as such the compounds are generally isolated as mixtures of the two compounds. Thus, references in the instant application to "a" compounds such as B1a, A1a and the like, are intended to define the pure compound as well as those which actually contain a certain proportion of the corresponding "b" compound. Alternatively, this representation of a mixture is sometimes done by referring to the B1 or B2 compounds or by separating the "a" compound from the "b" compound by a slash (/) such as B1a/B1b, B2a/B2b, and the like.

## PREPARATION OF STARTING MATERIALS

The ultimate starting materials for the compounds of this invention are the avermectin and milbemycin fermentation products defined above. Thus it is apparent that additional reactions are required to prepare many of the starting materials for the instant compounds. Specifically, reactions are carried out at the 4″, 4a, 5, 10, 13 and 23 positions. It is generally preferred to prepare whatever substituents are required at these positions before carrying out the reaction to introduce the fluoro or fluoro containing group on the substrate. Such a procedure generally avoids undesirable side reactions. This technique is not required, however, and if desired, other sequences may be used. In addition, it is often necessary to protect certain reactive hydroxy groups where reaction with the above reagents is not desired. With the appropriate positions protected, the above reactions may be carried out without affecting the remainder of the molecule. Subsequent to any of the above described reactions the protecting group may be removed and the unprotected product isolated. The protecting group employed is ideally one which may be readily synthesized, will not be affected by the reaction with fluorinating reagents and may be readily removed without affecting any other functions of the molecule. It is noted that certain instant protected compounds are novel and have considerable antiparasitic activity. They are included within the ambit of the instant invention. One preferred type of protecting group for the avermectin and milbemycin type of molecule is the tri-substituted silyl group, preferably the trialkyl silyl group. One especially preferred example is the t-butyl dimethylsilyl group. The reaction preparing the protected compound is carried out by reacting the hydroxy compound with the appropriately substituted silylhalide, preferably the silylchloride in an aprotic polar solvent such as dimethylformamide. Imidazole is added as a catalyst. The reaction is complete in from 1 to 24 hours at 0° to 25° C. For the 5-position hydroxy group the reaction is complete in from 1/2 to 3 hours at from 0° C to room temperature. This reaction is selective to the 5 position under the conditions above described and very little silylation is observed at other hydroxy groups. Alternatively, a 5, 13, 23-tri-(phenoxyacetyl) derivative can be prepared. Basic hydrolysis will leave the highly hindered 23-O-substituent but will hydrolyze the 5- and 13-O-phenoxy acetyl groups leaving them available for reaction. The 5-position may be selectively protected as described above with 5-butyldimethylsilyl, and the 13-hydroxy group may be reacted.

The silyl group may be removed after the other contemplated reactions have been carried out. The silyl group or groups are removed by stirring the silyl compound in methanol catalyzed by a catalytic amount of an acid, preferably a sulfonic acid such as p-toluenesulfonic acid. The reaction is complete in about 1 to 12 hours at from 0° to 50° C.

Another of the starting materials used in the foregoing reaction scheme are those in which the 22,23 double bond of the A1 and B1 compounds has been reduced to a single bond. As is readily apparent from

an analysis of the structure of avermectin starting materials there are 5 unsaturations in the 1-series of compounds. Thus in the "1" series of compounds it is necessary to reduce the 22,23 double bond while not affecting the remaining four unsaturations or any other functional group present on the molecule in order to selectively prepare the 22,23 dihydro avermectins. It is necessary to select a specific catalyst for the hydrogenation, one that will selectively hydrogenate the least hindered from among a series of unsaturations. The preferred catalyst for such a selective hydrogenation procedure is one having the formula:

$[(Ph_3P)_3RhZ)]$

wherein

Ph is phenyl and Z is halogen. The reduction procedure is completely described in U.S. Pat. No. 4,199,569 to Chabala et al.

Certain of the avermectin starting materials for the compounds of this invention require the removal of both of the a-L-oleandrosyl moieties (described in U.S. Pat. No. 4,206,205 to Mrozik et al.). The selective acylation of the susceptible hydroxy groups is described in U.S. Pat. No. 4,201,861 to Mrozik et al.

The reaction conditions which are generally applicable to the preparation of the aglycone involve dissolving the avermectin compound or the hydrogenated avermectin compound in an aqueous acidic non-nucleophilic organic solvent, miscible with water, preferably dioxane, tetrahydrofuran, dimethoxyethane, dimethylformamide, bis-2-methoxyethyl ether, and the like, in which the water concentration is from 0.1 to 20% by volume. Concentrated acid is added to the aqueous organic solvent to the extent of 1 to 10% by volume. The reaction mixture is generally stirred at about 20°-40° C, preferably at room temperature, for from 6 to 24 hours. The products are isolated, and mixtures are separated by techniques such as column, thin layer, preparative and high pressure liquid chromatography, and other known techniques.

The acids which may be employed in the above process include mineral acids and organic acids such as sulfuric, hydrohalic, phosphoric, trifluoroacetic, trifluoro methane sulfonic and the like. The hydrohalic acids are preferably hydrochloric or hydrobromic. The preferred acid in the above process is sulfuric acid.

A further procedure for the preparation of the aglycone of the avermectin compounds or of the hydrogenated avermectin compounds utilizes a different solvent system. For the preparation of the aglycone, 1% acid, by volume, in methanol under the foregoing reaction conditions has been found to be appropriate.

When this procedure is employed on the starting materials containing the 22,23-double bond, there is a possibility of an acid catalyzed addition of the solvent to the double bond. If such occurs, chromatographic purification will remove the by-product in order to allow for further reactions.

The acids listed above are appropriate for this process, and again sulfuric acid is the preferred acid.

The B1 and 22,23-dihydro B1 compounds have 2 available hydroxy groups: at the 4″-and the 5-positions. However, the two hydroxy groups have different reactivities. The 5-hydroxy group can be protected specifically by the preparation of the 5-O-tert-butyldimethylsilyl or other trisubstituted silyl derivative as described by Mrozik et al. in Tetrahedron Letters 24: 5333-5336 (1983).

The following examples are provided in order to more fully describe the present invention and are not to be construed as limitative of the invention.

The substituted avermectin derivatives prepared in the following examples are generally isolated as solids. They are characterized analytically using techniques such as mass spectrometry, nuclear magnetic resonance, and the like. The compounds are not characterized by sharp melting point; however, the chromatographic and analytical methods employed indicate that the compounds are of suitable purity.

In the following examples, the various starting materials therefore are avermectin compounds or derivatives of avermectin compounds. The avermectin compounds and the preparation and isolation thereof from fermentation broths are described in U.S. Pat. No. 4,310,519. The selective 22,23-dihydro derivatives of avermectin compounds are described in U.S. Pat. No. 4,199,569. The aglycone and monosaccharide derivatives of avermectin compounds are described in U.S. Pat. No. 4,206,205.

## EXAMPLE 1

### 4″-O-tert-Butyldimethylsilylavermectin B1a/B1b.

A solution of 1.0 g of 4″,5-di-O-tert butyldimethylsilylavermectin B1a/B1b in 50 ml of MeOH containing 0.5 g of p-toluenesulfonic acid was held at room temperature for 20 minutes. It was poured into dilute

aqueous sodium bicarbonate solution and extracted with ether. Silicagel column chromatography gave 380 mg of 4″-O-tert-butyldimethylsilylavermectin B1a/B1b, which was characterized by its mass and $^1$H-NMR spectra.

## EXAMPLE 2

4″-O-tert-Butyldimethylsilyl-5-deoxy-5-fluoroaver mectinB1a/B1b.

A solution 330 mg of 4″-O-tert-butyldimethylsilylavermectin B1a/B1b in 3.0 ml of $CH_2Cl_2$ was stirred under $N_2$ at -70° C, when a solution of 50 ml of diethylaminosulfur trifluoride (DAST) in 1.0 ml of $CH_2Cl_2$ was added. After 10 minutes the reaction mixture was poured into dilute aqueous sodium bicarbonate solution and extracted with ether. Purification by preparative silicagel layer chromato graphy gave 200 mg of 4″-O-tert-butyldimethyl-silyl-5-deoxy-5-fluoroavermectin B1a/B1b as an amorphous foam, which was characterized by its mass and NMR spectra.

## EXAMPLE 2a

5,13-Di-deoxy-5-fluoroavermectin B1a/B1b aglycone.

If 13-deoxyavermectin B1a/B1b aglycones are reacted according to the procedure fully described in example 2, 5,13-di-deoxy-5-fluoroavermectin B1a/B1b aglycones are obtained.

## EXAMPLE 2b

5-Deoxy-5-fluoromilbemycins $\alpha_1$ and/or $\alpha_3$.

If milbemycins $\alpha_1$ and/or $\alpha_3$ are submitted to the reaction conditions fully described in example 2, 5-deoxy-5 fluoromilbemycins $\alpha_1$ and/or $\alpha_3$ are obtained.

## EXAMPLE 2c

5,23-Di-deoxy-23,24-dehydro-5-fluoro-anthelmintic-F-28249-α.

If anthelmintic F-28249-α is reacted according to the procedure fully disclosed in example 2, but using an excess of reagent diethylaminosulfur trifluoride (DAST) with a reaction time of 30 minutes at -70° C, 5,23-di-deoxy-23,24-dehydro-5-fluoroanthelmintic-F-28249-α is obtained.

## EXAMPLE 3

5-Deoxy-5-fluoroavermectin B1a/B1b.

A solution of 200 mg of 4″-O-tert-butyldimethylsilylavermectin B1a/B1b in 3.0 ml of an anhydrous

hydrogen fluoride - pyridine - tetrahydrofuran mixture, prepared by mixing 14.0 ml of THF, 4.0 ml of pyridine and 2.0 ml of commercial HF - pyridine (consisting of 70% HF and 30% of pyridine, supplied by Aldrich Chemical Company) was kept at room temperature for two days. Then the reaction mixture was poured into dilute aqueous sodium bicarbonate solution and extracted with ether. Purification by preparative silicagel layer chromatography gave 122 mg of 5-deoxy-5-fluoroavermectin B1a/B1b as an amorphous foam, UV $\lambda_{max}$ (MeOH) 245 nm, E % 352, which was characterized by its mass and NMR spectra.

EXAMPLE 4

5-O-tert-Butyldimethylsilyl-4″,7-di-O-trimethylsilylavermectin B1a/B1b.

A solution of 50 mg of 5-O-tert-butyldimethylsilylavermectin B1a/B1b in 1.0 ml of anhydrous DMF was treated with 200 µL of bis(trimethylsilyl)trifluoroacetmide for 1 hour at room temperature. The reaction mixture was concentrated in high vacuum at room temperature and then purified via preparative silicagel layer chromatography to yield 50 mg of 5-O-tert-butyldimethylsilyl-4″,7-di-O-trimethylsilylavermectin B1a/B1b which was characterized by its mass and $^1H$ NMR spectra.

EXAMPLE 5

5-O-tert-Butyldimethylsilyl-7-O-trimethylsilylavermectin B1a/B1b.

A solution of 50 mg of 5-O-tert-butyldimethylsilyl-4″,7-di-O-trimethylsilylavermectin B1a/B1b in 1.0 ml of THF containing 10% water and 5% of p-toluenesulfonic acid was kept at room temperature for 20 minutes. Then the reaction mixture was poured into dilute aqueous sodium bicarbonate solution and extracted with ether. Purification by preparative silicagel layer chromatography gave 52 mg of 5-O-tert-butyldimethylsilyl-7-O-trimethylsilylavermectin B1a/B1b as an amorphous foam, which was characterized by its mass and NMR spectra.

EXAMPLE 6

5-O-tert-Butyldimethylsilyl-4″-deoxy-4″-fluoro-7-O-trimethylsilylavermectin B1a/B1b.

A solution of 52 mg of 5-O-tert-butyldimethylsilyl-7-O-trimethylsilylavermectin B1a/B1b in 1.5 ml of $CH_2Cl_2$ was stirred in an acetone-dry ice bath at -70° C. To this a solution of 10 µL of diethylaminosulfur trifluoride (DAST) in 0.5 ml of $CH_2Cl_2$ was added. The reaction mixture was kept for 15 minutes in the acetone-dry ice bath at -70° C, then allowed to warm to room temperature for two hours. The reaction mixture was worked up and the product isolated by addition to dilute aqueous sodium bicarbonate solution and extraction with ether. Purification by preparative silicagel layer chromatography gave 11 and 37 mg respectively of the two 4″-isomers of 5-O-tert-butyl-dimethylsilyl-4″-deoxy-4″-fluoro-7-O-trimethylsilylavermectin B1a/B1b as amorphous foams, which were characterized by their mass and NMR spectra.

EXAMPLE 7

4″-Deoxy-4″-fluoroavermectin B1a/B1b, isomer A.

11 Mg of 5-O-tert-butyldimethylsilyl-4″-deoxy-4″-fluoro-7-O-trimethylsilylavermectin B1a/B1b (isomer A)

is deprotected with 0.5 ml of an HF -pyridine - THF solution according to the procedure described in example 3. Isolation and purification by preparative silica gel layer chromatography gave 7 mg of 4″-deoxy-4″-fluoroavermectin B1a/B1b, isomer A, as amorphous foam, UV (MeOH) $\lambda_{max}$ 244 nm, E% 361, which was characterized by its mass and $^1$H-NMR spectra.

EXAMPLE 8

4″-Deoxy-4″fluoroavermectin B1a/B1b, isomer B.

37 Mg of 5-0-tert-butyldimethylsilyl-4″-deoxy-4″-fluoro-7-O-trimethylsilylavermectin B1a/B1b (isomer B) is deprotec ted according to the procedure of examples 3 and 7 to give 18 mg of pure 4″-deoxy-4″-fluoroavermectin B1a/B1b, isomer B, as amorphous foam, UV (MeOH) $\lambda_{max}$ 244 nm, E% 343, which was characterized by its mass and $^1$H-NMR spectra.

EXAMPLE 9

4″,5-Di-O-phenoxyacetylavermectin B1a/B1b.

A solution of 5.0 g of avermectin B1a and/or B1b, 2.24 g of 4-dimethylaminopyridine, 1.48 ml of anhydrous pyridine, in 75 ml of anhydrous $CH_2Cl_2$ stirred at 0° C is treated dropwise with 3.72 g (2.65 ml) of phenoxyacetyl chloride. The reaction mixture is stirred for an additional hour in an ice bath, then kept over night in a refrigerator. After an additional 4 hours of stirring at room temperature the reaction mixture is diluted with 200 ml of ether and washed in a separatory funnel with $H_2O$, aqueous $KH_2PO_4$, and $H_2O$, dried, and concentrated to a light yellow foam. Purification by silica gel column chromatography gave 6.42 g of 4″,5-di-O-phenoxyacetylavermectin B1a/B1b, which was characterized by its mass and $^1$H-NMR spectra.

EXAMPLE 9a

13-Deoxy-5-O-phenoxyacetylavermectin B1a/B1b aglycone.

If 13-deoxyavermectin B1a/B1b aglycones are reacted according to the procedure fully described in example 9, 13-deoxy-5-O-phenoxyacetylavermectin B1a/B1b aglycone is obtained.

EXAMPLE 9b

5-O-Phenoxyacetylmilbemycins $\alpha_1$ and/or $\alpha_3$.

If milbemycins $\alpha_1$ and/or $\alpha_3$ are submitted to the reaction conditions fully described in example 9, 5-O-phenoxyacetylmilbemycins $\alpha_1$ and/or $\alpha_3$ are obtained.

EXAMPLE 9c

5,23-Di-O-phenoxyacetyl-anthelmintic-F-28249-$\alpha$.

If anthelmintic F-28249-α is reacted according to the procedure fully disclosed in example 9, 5,23-di-O-phenoxyacetyl-anthelmintic-F-28249-α is obtained.

EXAMPLE 10

4a-Hydroxy-4″,5-di-O-phenoxyacetylavermectin B1a/B1b.

A reaction mixture containing 3.0 g of 4″,5 di-O-phenoxyacetylavermectin B1a/B1b, 650 mg of anhydrous $MgSO_4$, 146 mg of selenium dioxide ($SeO_2$), and 0.585 ml of tert-butyl hydroperoxide (90% solution) was stirred at room temperature for 21 hours. Then ice water was added to the reaction mixture, and the product was isolated by extraction with ether, washing the extract with $H_2O$, and concentration in vacuo. Purification by silicagel column chromatography gave 605 mg of 4a-hydroxy-4″,5-di-O-phenoxyacetylavermectin B1a/B1b, which was characterized by its mass and $^1$H-NMR spectra.

EXAMPLE 10a

4a-Hydroxy-13-deoxy-5-O-phenoxyacetylavermectin B1a/B1b aglycone.

If 13-deoxy-5-O-phenoxyacetylavermectin B1a/B1b aglycones are reacted according to the procedure fully described in example 10, 4a-hydroxy-13-deoxy-5-O-phenoxyacetylavermectin B1a/B1b aglycone is obtained.

EXAMPLE 10b

4a-Hydroxy-5-O-phenoxyacetylmilbemycins $\alpha_1$ and/or $\alpha_3$.

If 5-O-phenoxyacetylmilbemycins $\alpha_1$ and/or $\alpha_3$ are submitted to the reaction conditions fully described in example 10, 4a-hydroxy-5-O-phenoxyacetylmilbemycins $\alpha_1$ and/or $\alpha_3$ are obtained.

EXAMPLE 10c

4a-Hydroxy-5,23-di-O-phenoxyacetyl-anthelmintic-F-28249-α.

If 5,23-di-0 phenoxyacetyl-anthelmintic F-28249-α is reacted according to the procedure fully disclosed in example 10, 4a-hydroxy-5,23-di-O-phenoxyacetyl-anthelmintic-F-28249-α is obtained.

EXAMPLE 11

4a-Fluoro-4″,5-di-O-phenoxyacetylavermectin B1a/B1b.

A solution of 100 mg of 4a-hydroxy-4″,5-di-O-phenoxyacetylavermectin B1a/B1b in 510 µL of anhydrous $CH_2Cl_2$ was added to a solution of 13.7 µL of diethylaminosulfur trifluoride (DAST) in 510 µL of anhydrous $CH_2Cl_2$ stirred at -78°C (acetone-dry ice bath). After 30 minutes the acetone dry ice bath was

replaced with an acetone-ice bath for one hour. After 90 additional minutes at room temperature the reaction mixture was quenched with dilute aqueous $NaHCO_3$, and the product was isolated by ether extraction. Purification by preparative silicagel layer chromatography gave 56 mg of 4a-fluoro-4″,5-di-O-phenoxyacetylavermectin B1a/B1b, which was characterized by its mass and $^1$H-NMR spectra.

EXAMPLE 11a

4a-Fluoro-13-deoxy-5-O-phenoxyacetylavermectin B1a/B1b aglycone.

If 4a-hydroxy-13-deoxy-5-O-phenoxyacetylavermectin B1a/B1b aglycones are reacted according to the procedure fully described in example 11, 4a-fluoro-13-deoxy-5-O-phenoxyacetylavermectin B1a/B1b aglycones are obtained.

EXAMPLE 11b

4a-Fluoro-5-O-phenoxyacetylmilbemycins $\alpha_1$ and/or $\alpha_3$.

If 4a-hydroxy-5-O-phenoxyacetylmilbemycins $\alpha_1$ and/or $\alpha_3$ are submitted to the reaction conditions fully described in example 11, 4a-fluoro-5-O-phenoxyacetylmilbemycins $\alpha_1$ and/or $\alpha_3$ are obtained.

EXAMPLE 11c

4a-Fluoro-5,23-di-O-phenoxyacetylanthelmintic-F-28249-α.

If 4a-hydroxy-5,23-di-O-phenoxyacetylanthelmintic-F-28249-α is reacted according to the procedure fully disclosed in example 11, 4a-fluoro-5,23-di-O-phenoxyacetyl-anthelmintic-F-28249-α is obtained.

EXAMPLE 12

4a-Fluoro-22,23-dihydro-avermectin B1a/B1b.

A solution of 27 mg of 4a-fluoro-4″,5-di-O-phenoxyacetylavermectin B1a/B1b in 0.5 ml of anhydrous MeOH was treated with 80 µL of a methanolic sodium methoxide solution (prepared from 90 mg of sodium and 38 ml of anhydrous MeOH) for one hour at room temperature. The reaction mixture was worked up by addition of a drop of glacial acetic acid and concentrated under a stream of $N_2$. Preparative TLC, HPLC, mass and $^1$H-NMR spectral analyses of the crude product indicated a mixture consisting of 4a fluoroavermectin B1a/B1b, 2-epi-4a-fluoroavermectin B1a/B1b, 4″-O-phenoxyacetyl-4a-fluoroavermectin B1a/B1b, and 4″-O-phenoxyacetyl-2-epi-4a-fluoroavermectin B1a/B1b. Further purification of 34 mg of this and a similar crude reaction product was carried out by preparative reverse phase high performance liquid chromatography using a Whatman Partisil M20 10/50 ODS-3 column and gave 9 mg pure 4a-fluoroavermectin B1a, which was characterized by its mass and $^1$H-NMR spectra.

EXAMPLE 12a

4a-Fluoro-13-deoxyavermectin B1a/B1b aglycones.

If 4a-fluoro-13-deoxy-5-O-phenoxyacetylavermectin B1a/B1b aglycones are reacted according to the procedure fully described in example 12, 4a-fluoro-13-deoxyavermectin B1a/B1b aglycones are obtained.

EXAMPLE 12b

4a-fluoromilbemycins $\alpha_1$ and/or $\alpha_3$.

If 4a fluoro-5-O-phenoxyacetylmilbemycins $\alpha_1$ and/or $\alpha_3$ are submitted to the reaction conditions fully described in example 12, 4a-fluoromilbemycins $\alpha_1$ and/or $\alpha_3$ are obtained.

EXAMPLE 12c

4a-Fluoro-anthelmintic-F-28249-α.

If 4a-fluoro-5,23-di-O-phenoxyacetyl-F-28249-α is reacted according to the procedure fully disclosed in example 12, 4a-fluoro-anthelmintic-F-28249-α is obtained.

EXAMPLE 13a

5″-O-tert-Butyldimethylsilyl-11-bromo-10,11 dihydro-10-hydroxyavermectin B1a/B1b.

2.58 Grams of N-bromacetamide was added to a solution of 12 g of 5″-O-tert-butyldimethylsilylavermectin B1a/B1b in 180 ml of acetone and 20 ml of $H_2O$ and stirred at room temperature for 110 minutes, when silicagel thin layer chromatography indicated about one half of starting material was consumed. The reaction mixture was poured onto 400 ml $H_2O$ and the products extracted with ether. The ether extract was washed with aqueous $NaHCO_3$, dried and concentrated. The solid residue was purified by silicagel column chromatography (hexane-EtOAc 7:3, Waters Prep. 500) to give 5.2 g of 5″-O-tert-butyldimethylsilyl-11-bromo-10,11-dihydro-10-hydroxyavermectin B1a/B1b, which was characterized by its mass and $^1$H-NMR spectra.

EXAMPLE 13b

5″-O-tert-Butyldimethylsilyl-10,11-dihydro-10-hydroxyavermectin B1a/B1b.

A solution of 5.2 g of 5″-O-tert-butyldimethylsilyl-11-bromo-10,11-dihydro-10-hydroxyavermectin B1a/B1b in 6 ml of toluene and 6.0 ml of tributyltin hydride was heated at 105° C for 3 hours under $N_2$. The reaction mixture was allowed to come to room temperature and purified by silicagel column chromatography (hexane-EtOAC 2:1, Waters Prep. 500) to give 4.0 g of 5″-O-tert-butyldimethylsilyl-10,11-dihydro-10-hydroxyavermectin B1a/B1b, which was characterized by its mass, $^1$H-, and $^{13}$C-NMR spectra.

EXAMPLE 13c

5″-O-tert-Butyldimethylsilyl-4″-deoxy-10,11-dihydro-4″,10-difluoroavermectin B1a/B1b.

A solution of 302 mg of 5″-O-tert-butyldimethylsilyl-10,11-dihydro-10-hydroxyavermectin B1a/B1b in 3.0 ml of dry $CH_2Cl_2$ cooled to -78° C was treated with 81 µL of diethylaminosulfur trifluoride (DAST) dropwise from a syringe. The reaction mixture was stirred 1.5 hours at -78° C, 1 hour at -20° C, and 1 hour at room temperature. Then the reaction mixture was poured onto dilute aqueous $NaHCO_3$, extracted with $CH_2Cl_2$, dried over $MgSO_4$, and concentrated. The residue was purified by silicagel column chromatography and then separated by preparative reverse phase high performance liquid chromatography into 76 and 26 mg respectivly of the two 4″-isomers A and B of 5″-O-tert-butyldimethylsilyl-4″-deoxy-10,11-dihydro-4″,10-difluoroavermectin B1a/Blb, which were characterized by their mass and $^1$H-NMR spectra.

EXAMPLE 13d

4″-Deoxy-10,11-dihydro-4″,10-difluoroavermectin B1a/B1b, 4″-Isomer A.

A solution of 76 mg of 5″-O-tert-butyldimethylsilyl-4″-deoxy-10,11-dihydro-4″10-difluoroavermectin B1a/B1b-Isomer A in 3.0 ml of a HF -pyridine - THF - mixture was treated and isolated as fully described in example 3 to give 48 mg of 4″-deoxy-10,11-dihydro-4″,10-difluoroavermectin B1a/B1b, 4″-Isomer A, which was characterized by its mass and $^1$H-NMR spectra.

EXAMPLE 13e

4″-Deoxy-10,11-dihydro-4″,10-difluoroavermectin B1a/B1b, 4″-Isomer B.

A solution of 24 mg of 5″-O-tert-butyldimethylsilyl-4″-deoxy-10,11-dihydro-4″,10-difluoro-avermectin B1a/B1b-Isomer B in 1.2 ml of a HF -puridine - THF - mixture was treated and isolated as fully described in example 3 to give 17 mg of 4″-deoxy-10,11-dihydro-4″,10-difluroravermectin B1a/B1b, 4″-Isomer B, which was characterized by its mass and $^1$H-NMR spectra.

EXAMPLE 14

5,13-Difluoro-22,23-dihydroavermectin B1a/B1b aglycone.

A solution of 1.0 g of 22,23-dihydroavermectin B1a/B1b aglycone in 8 ml of $CH_2Cl_2$ is added dropwise from a syringe to a solution of 0.2 ml of diethylaminosulfur trifluoride (DAST) in 8 ml of $CH_2Cl_2$ stirred at -70° C under $N_2$. After 60 minutes at this temperature the reaction mixture is poured into aqueous $NaHCO_3$, and the products are extracted with $CH_2Cl_2$. Purification by preparative silicagel layer chromatography gives 5,13-difluoro-22,23-dihydroavermectin B1a/B1b aglycone, which is characterized by its mass and $^1$H-NMR spectra.

EXAMPLE 15a

5-O-tert-Butyldimethylsilyl-4a-fluoroavermectin B1a/B1b.

A solution of 5 g of 4a-fluoroavermectin B1a/B1b, 2.4 g of imidazole and 2.4 g of tert-butyldimethylsilyl chloride in 40 ml of anhydrous DMF is stirred at room temperature for 50 minutes. Then the reaction mixture is poured into 150 ml of ice cold water and the aqueous phase is extracated four times with 20 ml of ether. The organic phase is washed twice with water, aqueous NaCl solution, dried with $MgSO_4$ and concentrated in vacuo to a white foam. The crude product is purified by silicagel column chromatography with a $CH_2Cl_2$-EtOAC-90:10 to 70:30 solvent system to give 5 -O-t-butyldimethylsilyl-4a-fluoroavermectin B1a/B1b as an amorphous foam, which is characterized by its mass and $^1$H-NMR spectra.

## EXAMPLE 15b

5-O-tert-Butyldimethylsilyl-4a-fluoro-4″-oxoavermectin B1a/B1b.

To a solution containing 0.91 ml of oxalyl chloride in 23 ml of dry $CH_2Cl_2$ stirred at -60° C is added 1.5 ml of dry dimethylsulfoxide dissolved in 12 ml of dry $CH_2Cl_2$ during 15 minutes. Then a solution of 4.65 g of 5-O-t-butyldimethylsilyl-4a-fluoroavermectin B1a/B1b dissolved in 23 ml of dry $CH_2Cl_2$ is added over a period of 15 minutes while maintaining the temperature at -60° C. The reaction mixture is stirred at this temperature for 30 minutes when 6.5 ml of dry triethylamine is added. The mixture is stirred for 5 additional minutes at -60° C, and then the cooling bath is removed and the reaction mixture is allowed to come to ambient temperature. After addition of water the reaction product is extracted with $CH_2Cl_2$, the extract is washed with water, dried and concentrated in vacuo to a yellow foam. This is identified by its mass and NMR spectra as 5-O-t-butyldimethylsilyl-4a-fluoro-4″-oxoavermectin B1a/B1b, which is used for further chemical reactions without purification.

## EXAMPLE 15c

4″-Deoxy-4″-epi-methylamino-5-O-tert-butyldimethylsilyl-4a-fluoroavermectin B1a/B1b.

A solution of 2.6 ml of glacial acetic acid in 30 ml of MeOH is treated with methylamine gas at 0° C until the pH of the solution reaches 9.0. To this a solution containing 4.45 g of 5-O-t-butyldimethylsilyl-4a-fluoro-4″-oxoavermectin B1a/B1b in 20 ml of MeOH is added, the reaction mixture is stirred at room temperature for 1 hour, when a solution of 0.35 g of sodiumcyanoborohydride in 7.5 ml of MeOH is added dropwise over 10 minutes. After 50 minutes the reaction mixture is poured into 0.15 l of cold aqueous $Na_2CO_3$ solution and the product is extracted with ether. The extract is washed with water, dried, and concentrated in vacuo to a yellow foam. Thin layer chromatography (silica gel, $CH_2Cl_2$-EtOAc 85:15) of the crude product at this point shows several spots. Further purification by silica gel column chromatography using $CH_2Cl_2$-EtOAc solvent mixtures gives 5-O-t-butyldimethylsilyl-4a-fluoro-4″-epi-avermectin B1a/B1b, 5-O-t-butyldimethylsilyl-4a-fluoro-4″-deoxy-N-methylaminoavermectin B1a/B1b, and as the major product 5-O-t-butyldimethylsilyl-4a-fluoro-4″-deoxy-4″-epi-N-methylaminoavermectin B1 as light foams, which are characterized by their mass and $^1$H-NMR spectra.

## EXAMPLE 15d

4a-Fluoro-4″-deoxy-4″-epi-methylaminoavermectin B1a/B1b.

A solution of 1.4 g of 4a-fluoro-4″-deoxy-4″-epi-methylamino-5-O-t-butyldimethylsilylavermectin B1a/B1b in 20 ml of MeOH and a solution of 0.7 g of p-toluenesulfonic acid monohydrate in 50 ml of MeOH is mixed and stirred at room temperature for 45 minutes, and then poured into dilute aqueous $Na_2CO_3$ solution. The product is extracted with EtOAC, washed with water and dried over $MgSO_4$, concentrated in vacuo, and purified by preparative silicagel column chromatography with a $CH_2Cl_2$-MeOH 95:5 solvent mixture to give

4a-fluoro-4″-deoxy-4″-epi-methylaminoavermectin B1a/B1b, which is identified by mass $^1$H-NMR spectra.

EXAMPLE 15e

5-Deoxy-5-fluoro-4″-oxoavermectin B1a/B1b.

When 5-deoxy-5-fluoroavermectin B1a/B1b obtained in example 3 is treated according to the procedure fully described in Example 15b 5-deoxy-5-fluoro-4″-oxoavermectin B1a/B1b is obtained, which is characterized by its mass and $^1$H-NMR spectra.

EXAMPLE 15f

4″,5-Dideoxy-5-fluoro-4″-epi-methylaminoavermectin B1a/B1b.

When 5-deoxy-5-fluoro-4″-oxoavermectin B1a/B1b obtained in example 15e is treated according to the procedure fully described in example 15c 4″,5-dideoxy-5-fluoro-4″-epi-methylaminoavermectin B1a/B1b is obtained, which is characterized by its mass and $^1$H-NMR spectra.

EXAMPLE 16a

4″,5-Di-O-tert-butyldimethylsilylavermectin B2a/B2b.

A solution of 1.0 g of avermectin B2a/B2b, 0.614 g of imidazole, 0.680 mg of tert-butyldimethylsilyl chloride in 12.8 ml of anhydrous DMF was stirred at room temperature under $N_2$ for 20.5 hours. Then water and ether were added, the organic phase separated, washed repeatedly with water and concentrated in vacuo. Purification by silicagel column chromatography gave 401 mg of 4″,5,23-tri-O-tert-butyldimethylsilyl-avermectin B2a/B2b, 437 mg of the desired 4″,5-di-O-tert-butyldimethylsilylavermectin B2a/B2b, 115 mg of 5,23-di-O-tert-butyldimethylsilyl-avermectin B2a/B2b, and 129 mg of 5-O-tert-butyldimethylsilyl-avermectin B2a/B2b, all of which were characterized by their mass and/or $^1$H-NMR spectra.

EXAMPLE 16b

4″-O-tert-Butyldimethylsilylavermectin B2a/B2b.

A solution of 250 mg of 4″,5-di-O-tert-butyldimethylsilylavermectin B2a/B2b in 12.5 ml of MeOH containing 0.125 g of p-toluenesulfonic acid monohydrate is held at room temperature for 20 minutes. It is poured into dilute aqueous sodium bicarbonate solution and extracted with ether. Silicagel column chromatography gives pure 4″-O-tert-butyldimethylsilylavermectin B2a/B2b, which is characterized by its mass and $^1$H-NMR spectra.

EXAMPLE 16c

4″-O-tert-Butyldimethylsilyl-23,24-dehydro-5,23-di-deoxy-5-fluoroavermectin B2a/B2b.

A solution of 100 mg of 4″-O-tert butyl dimethylsilylavermectin B2a/B2b in 1.0 ml of $CH_2Cl_2$ is stirred under $N_2$ at -70° C, when a solution of 50 µL of diethylaminosulfur trifluoride (DAST) in 1.0 ml of $CH_2Cl_2$ is added. After 30 minutes the reaction mixture is poured into dilute aqueous sodium bicarbonate solution and extracted with ether. Purification by preparative silicagel layer chromatography gives 4″-O-tert-butyldimethylsilyl-23,24-dehydro-5,23-di-deoxy-5-fluoroavermectin B2a/B2b as an amorphous foam, which is characterized by its mass and $^1H$-NMR spectra.

## EXAMPLE 16d

23,24-Dehydro-5,23-di-deoxy-5-fluoroavermectin B2a/B2b.

If a solution of 50 mg of 4″-O-tert-butyldimethylsilyl-23,24-dehydro-5,23-di-deoxy-5-fluoroavermectin B2a/B2b in 1.0 ml of an anhydrous hydrogen fluoride - pyridine - tetrahydrofuran mixture as fully described in example 3 is held at room temperature for two days, one obtains 23,24-dehydro-5,23-di-deoxy-5-fluoroavermectin B2a/B2b, which is characterized by its mass and $^1H$-NMR spectra.

## EXAMPLE 16e

4″-Deoxy-4″-fluoroavermectin B2a/B2b.

If 115 mg of 5,23-di-O-tert-butyldimethylsilylavermectin B2a/B2b obtained in example 16a, are reacted with diethylaminosulfur trifluoride (DAST) as fully described in example 16c, and its protecting groups are removed as fully described in example 16d one obtains 4″-deoxy-4″-fluoroavermectin B2a/B2b, which is characterized by its mass and $^1H$-NMR spectra.

## EXAMPLE 16f

23,24-Dehydro-4″,23-dideoxy-4″-fluoroavermectin B2a/B2b.

If 129 mg of 5-O-tert butyldimethylsilylavermectin B2a/B2b, obtained in example 16a, are reacted with diethylaminosulfur trifluoride (DAST) as fully described in example 16c, and its protecting group is remvoed as fully descried in example 16d, one obtains 23,24-dehydro-4″,23-dideoxy-4″-fluoroavermectin B2a/B2b, which is characterized by its mass and $^1H$-NMR spectra.

## Claims

1. A compound having the formula:

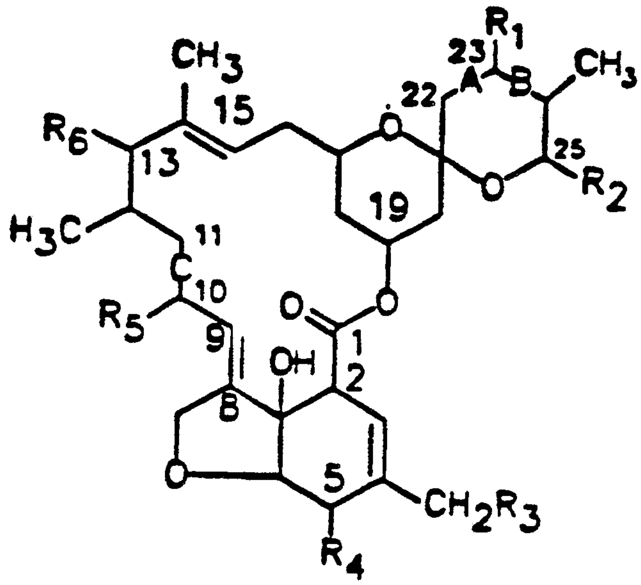

wherein
A, B and C indicate single or double bonds;
$R_1$ is hydrogen, or hydroxy when A and B are single bonds;
$R_2$ is $CH_3$, $C_2H_5$, $CH(CH_3)_2$, $CH(CH_3)C_2H_5$, $C(CH_3)=CHCH_3$, $C(CH_3)=CHC_2H_5$, $C(CH_3)=CHCH(CH_3)_2$;
$R_3$ is hydrogen or fluorine;
$R_4$ is hydroxy or fluorine;
$R_5$ is hydrogen, or fluorine when C is a single bond;
$R_6$ is hydrogen, hydroxy, chlorine, fluorine or

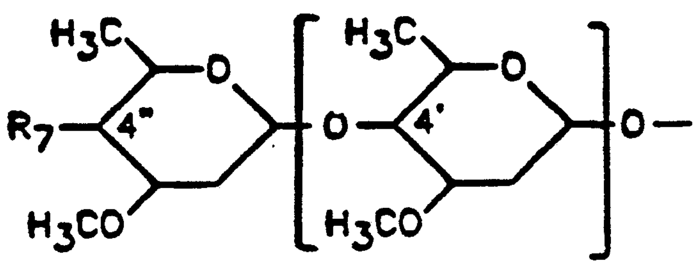

n=1 or 0

wherein
$R_7$ is hydroxy, fluorine, $NH_2$, NH-alkyl, N,N-dialkyl, NHCO-alkyl, N(alkyl)CO-alkyl and n is 0 or 1, provided that at least one group of $R_3$, $R_4$ or $R_7$ is a fluoro group.

2. A compound of claim 1 which is 5-deoxy-5-fluoroavermectin B1a/B1b.

3. A compound of claim 1 which is 5,13-Di-deoxy-5-fluoro-22,23-dihydroavermectin B1a/B1b aglycone.

4. A compound of claim 1 which is 5-Deoxy-5-fluoromilbemycins $\alpha_1$ and/or $\alpha_3$.

5. A compound of claim 1 which is 5,23-Di-deoxy-23,24-dehydro-5-fluoro-anthelmintic-F-28249-$\alpha$.

6. The use of a compound of Claim 1 for the preparation of a composition useful for the treatment of parasitic infections.

7. A composition useful for treating animals infected and an effective amount of a compound of Claim 1.

8. A method for the treatment of household or agricultural insect pests which comprises applying to an area infested with such insect pests, an effective amount of a compound of claim 1.

9. A composition useful for treating areas infected with household or agricultural insect pests which comprise an inert carrier and effective amount of a compound of claim 1.

10. A process for preparing the compounds of claim 1, which comprises treating an avermectin compound unprotected in the 4a, 5, or 4″ position with diethylaminosulfur trifluoride (DAST).